(19) Europäisches Patentamt
European Patent Office
Office européen des brevets

(11) Publication number: **0 085 440**
**B1**

(12) **EUROPEAN PATENT SPECIFICATION**

(45) Date of publication of patent specification: **09.10.85**

(51) Int. Cl.[4]: **C 07 H 19/14**

(21) Application number: **83101511.0**

(22) Date of filing: **25.01.82**

(60) Publication number of the earlier application in accordance with Art. 76 EPC: **0 057 548**

(54) **Production of 4-chloro-7-(2,3,5-tri-O-benzyl-beta-D-arabinofuranosyl)-7H-pyrrolo(2,3-d) pyrimidine compounds.**

(30) Priority: **29.01.81 US 229471**

(43) Date of publication of application: **10.08.83 Bulletin 83/32**

(45) Publication of the grant of the patent: **09.10.85 Bulletin 85/41**

(84) Designated Contracting States: **AT BE CH DE FR GB IT LI LU NL SE**

(56) References cited:
**CHEMISCHE BERICHTE, vol. 113, 1980, pages 2069-2080, Verlag Chemie GmbH, Weinheim, DE., H.D. WINKELER et al.: "4-Amino-7-(beta-D-arabinofuranosyl)pyrrolo(2,3-d)pyrimidin - die Synthese von Ara-Tubercidin durch Phasentransferkatalyse"**

**CHEMISCHE BERICHTE, vol. 112, no. 10, 1979, pages 3432-3440, Verlag Chemie GmbH, Weinheim, DE., U. LÜPKE et al.: "7-(beta-D-Arabinofuranosyl)pyrrolo(2,3-d)pyrimidin-4(3H)-on - das 7-Desazaderivat des antiviralen Nucleosids Ara-H"**

**Agric. Biol. Chem., Vol. 47 (1977), pp. 1501-1507**

(73) Proprietor: **WARNER-LAMBERT COMPANY**
**201 Tabor Road**
**Morris Plains New Jersey 07950 (US)**

(72) Inventor: **Cook, Philip Daniel**
**2333 Georgetown Boulevard**
**Ann Arbor Michigan 48105 (US)**

(74) Representative: **Jones, Michael Raymond et al**
**HASELTINE LAKE & CO. Hazlitt House 28**
**Southampton Buildings Chancery Lane**
**London WC2A 1AT (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to a process for producing two particular 4-chloro-7-(2,3,5-tri-O-benzyl-β-D-arabinofuranosyl)-7H-pyrrolo[2,3-d]pyrimidine compounds.

The production of the 2-methylmercapto derivative by a two-phase reaction between 2,3,5-tri-O-benzyl-D-arabinofuranosyl bromide and 4-chloro-2-methylmercapto-7H-pyrrolo[2,3-d]pyrimidine is known from Chem. Ber. Vol. 113 (1980) pp. 2069—2080. The reaction was carried out in benzene/aqueous NaOH using benzyl triethyl ammonium chloride as phase-transfer catalyst.

According to the present invention, there is provided a process for producing a 4-chloro-7-(2,3,5-tri-O-benzyl-β-D-arabinofuranosyl)-7H-pyrrolo[2,3-d]pyrimidine compound having the general formula:—

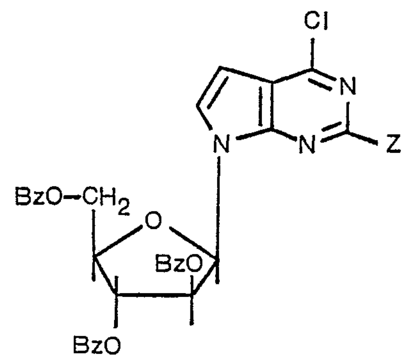

wherein Z is a hydrogen atom or a methylmercapto group and Bz represents a benzyl radical, which process comprises treating 4-chloro-7H-pyrrolo[2,3-d]pyrimidine or the corresponding 2-methylmercapto compound with sodium hydride in an polar solvent to form an anion having the formula:

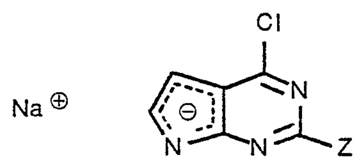

and β-D-arabinofuranosylating that anion with 2,3,5-tri-O-benzyl-α-D-arabinofuranosyl chloride.

Thus, a compound can be produced in accordance with the present invention by first treating the corresponding 4-chloro-7H-pyrrolo[2,3-d]pyrimidine with sodium hydride in a suitable solvent to form the anion having the formula:—

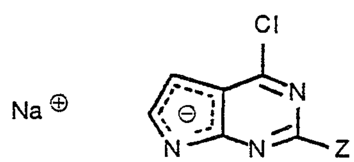

and glycosylating that anion with 2,3,5-tri-O-benzyl-α-D-arabinofuranosyl chloride.

The present, novel coupling process overcomes the difficulties and shortcomings prominent in the prior art. A preformed anion of the heterocycle, preferably in dimethylformamide (DMF), is utilised to increase nucleophilicity and solubility. The chloride becomes associated with the sodium to form sodium chloride. Coupling may be completed at ambient temperatures, usually within 0.5 hour. What was unpredicted in this process was that, although a very polar solvent was used, which would tend to favour sugar anomerization and thus approximately equal amounts of the α and β nucleosides would be expected, coupling under the above-mentioned conditions was rapid and preferentially took place before significant anomerization occurred. This relatively simple process provided high yields of the β-nucleoside. Anion formation of the requisite heterocycles may be obtained by treating the heterocycle dissolved in dry DMF with sodium hydride. The anion formation generally requires approximately 10—15 minutes rather than 12—20 hours of reflux in hexamethyldisilazane or acetonitrile and bissilytrifluoroacetamide and then distillation to obtain a silylated heterocycle. Other anion formation procedures employing different polar solvents may be used; however, sodium hydride in DMF is preferred owing to its experimental simplicity.

The use of anionic heterocycles in DMF or other polar solvents greatly increases the nucleophilicity of the heterocycle and allows the use of heterocycles possessing a variety of substituents. This latter advantage is particularly important since proper choice and positioning of substituents may be utilized to direct the arabinofuranosyl group to the desired site in the heterocycle. A polar solvent such as DMF is essential to solubilize the anionic heterocycle.

The resulting blocked 4-chloronucleosides produced in accordance with the present invention are

useful as intermediates in the preparation of antiviral agents, as described in European Patent Application No. 82300366.0 (EP—0057548—A2).

As to the starting materials, the synthesis of 4-chloro-7H-pyrrolo[2,3-d]-pyrimidine is described by Dovoll in J. Chem. Soc., 131 (1960). The synthesis of 4-chloro-2-methylmercapto-7H-pyrrolo[2,3-d]pyrimidine is described by C. W. Noell and R. K. Robins in J. Heterocyclic Chem., *1*, 34 (1964). The requisite sugar, 2,3,5-tri-O-benzyl-α-D-arabinofuranosyl chloride is described by C. P. J. Glaudemans, et al., J. Am. Chem. Soc., 87, 4636 (1965).

The invention will now be illustrated by the following Examples.

Example 1

4-Chloro-7-(2,3,5-tri-O-benzyl-β-D-arabinofuranosyl)-7H-pyrrolo[2,3-d]pyrimidine.

A suspension of 240 mg of sodium hydride, 1.54 g of 4-chloro-7H-pyrrolo[2,3-d]pyrimidine, and 6 ml of dry dimethylformamide (DMF) is stirred at 50°C for 15 minutes, cooled, and added to 4.83 g of 2,3,5-tri-O-benzyl-α-D-arabinofuranosyl chloride in 4 ml of DMF. The solution is stirred at ambient temperature for two hours, concentrated *in vacuo*, and distributed between a mixture of ethyl acetate-water. Chromatography of the dried ethyl acetate layer over silica gel with 4:1 benzene-ethyl acetate provides 3.3 g of yellow, syrupy 4-chloro-7-(2,3,5-tri-O-benzyl-β-D-arabinofuranosyl)-7H-pyrrolo[2,3-d]pyrimidine.

Example 2

4-Chloro-2-methylmercapto-7-(2,3,5-tri-O-benzyl-β-D-arabinofuranosyl)-7H-pyrrolo[2,3-d]pyrimidine.

A suspension of 3.1 g sodium hydride, 25.9 g of 4-chloro-2-methylmercapto-7H-pyrrolo[2,3-d]pyrimidine, and 250 ml of dry dimethylformamide (DMF) is stirred at 50°C for 0.5 hour, cooled, and added to 62.8 g of 2,3,5-tri-O-benzyl-α-D-arabinofuranosyl chloride. The solution is stirred at ambient temperature for 15 hours, concentrated *in vacuo*, and distributed between a mixture of ethyl acetate and water. Chromatography of the dried ($MgSO_4$) layer over silica gel with 4:1 toluene-ethyl acetate provides 76 g of yellow, syrupy 4-chloro-2-methylmercapto-7-(2,3,5-tri-O-benzyl-β-D-arabinofuranosyl)-7H-pyrrolo[2,3-d]pyrimidine.

**Claim**

A process for producing a 4-chloro-7-(2,3,5-tri-O-benzyl-β-D-arabinofuranosyl)-7H-pyrrolo[2,3-d]pyrimidine compound having the general formula:—

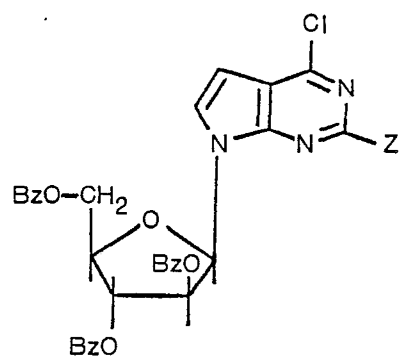

wherein Z is a hydrogen atom or a methylmercapto group and Bz represents a benzyl radical, which process comprises treating 4-chloro-7H-pyrrolo[2,3-d]pyrimidine or the corresponding 2-methylmercapto compound with sodium hydride in a polar solvent to form an anion having the formula:

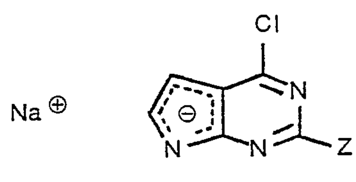

and β-D-arabinofuranosylating that anion with 2,3,5-tri-O-benzyl-α-D-arabinofuranosyl chloride.

**Patentanspruch**

Verfahren zur Herstellung einer 4-Chlor-7-(2,3,5-tri-O-benzyl-β-D-arabinofuranosyl)-7H-pyrrolo[2,3-d]pyrimidin-Verbindung der allgemeinen Formel

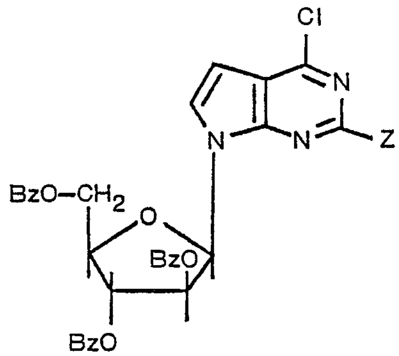

worin Z ein Wasserstoffatom oder eine Methylmercaptogruppe ist und Bz ein Benzylradikal darstellt, welches Verfahren dadurch gekennzeichnet ist, daß 4-Chlor-7H-pyrrolo[2,3-d]pyrimidin oder die entsprechende 2-Methylmercaptoverbindung mit Natriumhydrid in einem polaren Lösungsmittel behandelt wird, um ein Anion der Formel

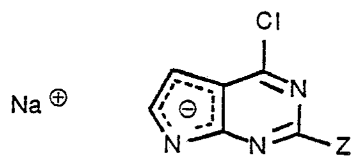

zu bilden, und daß dieses Anion mit 2,3,5-Tri-O-benzyl-α-D-arabinofuranosylchlorid β-D-arabinofuranosyliert wird.

**Revendication**

Un procédé de préparation d'un dérivé de 4-chloro-7-(2,3,5-tri-O-benzyl-β-D-arabinofuranosyl)-7H-pyrrolo[2,3-d]pyrimidine de formule générale:

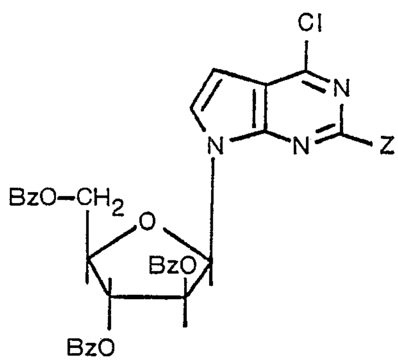

dans laquelle:

Z représente un atome d'hydrogène ou un groupe méthylmercapto; et

Bz représente un radical benzyle,

ce procédé consistant à traiter une 4-chloro-7H-pyrrolo[2,3-d]-pyrimidine ou le dérivé de 2-méthylmercapto correspondant par de l'hydrure de sodium dans un solvant polaire pour former un anion de formule:

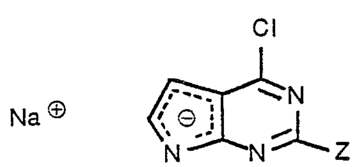

et à procéder à la β-D-arabinofuranosylation de cet anion par le chlorure de 2,3,5-tri-O-benzyl-α-D-arabinofuranosyle.